# EUROPEAN PATENT SPECIFICATION

(11) **EP 2 575 953 B1**
(45) Date of publication and mention of the grant of the patent: **11.07.2018**
(21) Application number: 11724885.6
(22) Date of filing: 26.05.2011
(51) Int. Cl.: A61M 39/20, A61M 5/38, A61M 5/14

(54) **CONNECTOR CAP FOR PRIMING A TRANSFUSION LINE**
VERSCHLUSSKAPPE ZUM FÜLLEN UND ENTLÜFTEN EINES INFUSIONSSCHLAUCHS
CAPUCHON DE CONNECTEUR POUR L'AMORÇAGE D'UN TUYAU DE TRANSFUSION

(30) Priority: 27.05.2010 JP 2010121570
(43) Date of publication of application: 10.04.2013
(73) Proprietor: KPR U.S., LLC, Mansfield, MA 02048 (US)
(72) Inventor: MIYASAKA, Susumu, Tokyo (JP)
(74) Representative: Prock, Thomas
(86) International application number: PCT/US2011/038100
(87) International publication number: WO 2011/150184

(56) References cited:
- EP-A1- 0 462 702
- WO-A2-2004/091688
- US-A1- 2007 156 118

## Description

### BACKGROUND

### 1. Technical Field

The present disclosure relates to a connector cap used in priming of a transfusion line which conducts a fluid such as liquid medicine to a patient, and a transfusion line connection apparatus for use with the connector cap.

### 2. Background of Related Art

In recent years, so-called closed-loop transfusion systems which function to prevent infection and accidental needle sticking have come to be used in medical transfusion lines. Closed-loop transfusion systems comprise a catheter joint made up of a male connector and a female connector, for example. A seal such as a septum is provided on the female connector side, whereby a fluid such as liquid medicine is sealed. When the male luer of the male connector is connected to the seal of the female connector, the fluid such as liquid medicine can flow through the connector. Such a closed-loop transfusion system can be found in Japanese Unexamined Patent Application Publication No. H8-500983 ("JP H8-500983").

When a transfusion system having a joint part, as described in JP H8-500983, is primed, there are cases where the fluid, such as, for example, an anti-cancer agent or transfusion agent, leaks from the opening on the end of the male luer of the male connector. Such fluids may cause cell damage or birth defects, or may be genotoxic or carcinogenic. Thus, there is a desire for a transfusion system in which fluid does not leak when the system is primed and which prevents the health care provider from coming in contact with the fluid. A connector cap comprising a "storage apparatus having a first opening on the proximal end, and a second opening in which a hydrophobic filter is arranged" was proposed in Japanese Unexamined Patent Publication No. 2009-522048 ("JP 2009-522048") with the objective of preventing leakage during priming.

The connector cap disclosed in JP 2009-522048 is used by being attached to the male connector during priming. Thus, during priming, air passes through the hydrophobic filter of the connector cap to the outside, but liquid that has flowed out from the opening in the male luer passes through the first opening of the connector cap and is held inside the storage apparatus.

However, after priming is finished, when the connector cap is detached from the male connector, there is risk of the liquid that flowed into the storage apparatus (container) of the connector cap squirting out from the first opening, being aerated or leaking out. US 2007/156118 discloses a protective priming cap for a self-sealing male luer according to the preamble of claim 1.

The present disclosure resolves the above problems, and provides a connector cap which suppresses fluid leakage and a transfusion line connection apparatus for use with the connector cap.

### SUMMARY

Disclosed herein is a connector cap which can be removably attached to a male connector, including a container having a first opening and a second opening; a seal member, which is disposed in the first opening so as to separate the inside and outside of the container, the seal member including an insertion passage formed therethrough whereby an inserted body can be inserted from the outside to the inside of the container, the seal member having an elastic force by which ease of insertion of the inserted body and sealabilty after removal of the inserted body are both achieved; and a hydrophobic filter which is disposed in the second opening.

The connector cap may include a cover which can cover the second opening in a liquid-tight state from the outside of the filter.

The connector cap may include a filter attachment which removably attaches the filter to the second opening.

Also disclosed is a transfusion line connection apparatus including the connector cap and a male connector having an injection tube which can be inserted into the insertion passage of the seal member of the connector cap.

The connector cap being as described above. For this reason, fluid that flowed inside the container during priming can be sealed inside the container, even if the connector cap is removed from the male connector. Therefore, leakage during priming can be prevented.

The connector cap includes a cover which can cover the filter of the second opening in a liquid-tight state from the outside. For this reason, in the event that the hydrophobic filter becomes hydrophilic, leakage of fluid via the hydrophobic filter can be suppressed. Also, by covering the filter with the cover after priming, air can be prevented from being sucked into the male connector via the filter after priming.

The connector cap includes a filter attachment which removably attaches the hydrophobic filter to the second opening. For this reason, in the event that the hydrophobic filter becomes hydrophilic, priming can be performed after replacing only the filter.

Because the transfusion line connection apparatus disclosed herein includes the connector cap described above, leaking during priming can be prevented.

### BRIEF DESCRIPTION OF THE DRAWINGS

Various embodiments of the present disclosure are described hereinbelow with references to the drawings, wherein:
FIG. 1 is a schematic diagram illustrating the configuration of a transfusion line set according to embodiment 1;
FIGS. 2A-2C are diagrams showing a plan view, side view and vertical cross-sectional view of a transfusion line connection apparatus according to embodiment 1;
FIGS. 3A-3D are diagrams showing an elevation view, side view, rear view and vertical cross-sectional view of a connector cap according to embodiment 1;
FIG. 4 is a schematic cross-sectional diagram illustrating the connected state of a male connector and connector cap according to embodiment 1;
FIGS. 5A-5B are diagrams showing a plan view and side cross-sectional view of a transfusion line connection apparatus according to embodiment 2;
FIGS. 6A-6D are diagrams showing an elevation view, side view, rear view and vertical cross-sectional view of a connector cap according to embodiment 2;
FIG. 7 is a schematic cross-sectional diagram illustrating the connected state of a male connector and connector cap according to embodiment 2; and
FIGS. 8A-8B are cross-sectional diagrams illustrating a transfusion line connection apparatus according to embodiment 3.

### DETAILED DESCRIPTION OF THE EMBODIMENTS

In the drawings and in the description which follows like references numerals identify similar or identical elements.

### Embodiment 1

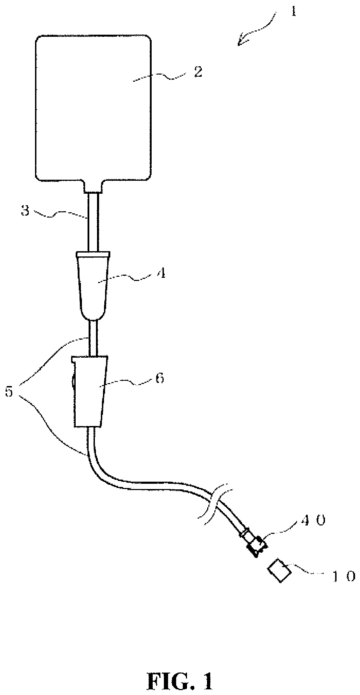

FIG. 1 is a diagram illustrating an example of the configuration of a transfusion line set according to embodiment 1.

A transfusion line set 1 has the purpose of supplying liquid medicine or the like, and includes a fluid container 2 in which fluid such as liquid medicine is placed, a drip cylinder 4, a roller clamp 6, a male connector 40 and a connector cap 10.

The fluid container 2 is a container which stores various medical liquids, such as liquid medicines, nutrients, anti-cancer agents, physiological saline solution, blood and so forth.

The drip cylinder 4 temporarily holds the liquid medicine or the like supplied from the fluid container 2 via a tube 3, and it sends the liquid medicine or the like, a specified quantity at a time, to the roller clamp 6 via a tube 5.

The roller clamp 6 is for adjusting the quantity of fluid such as liquid medicine supplied via the tube 5. The roller clamp 6 can adjust the fluid quantity by compressing the flexible tube 5, and can stop the flow of liquid medicine or the like when the tube 5 is compressed to the maximum limit.

The male connector 40 is attached to the end on the downstream side of the tube 5. A joint part for medical use is constructed by connecting the male connector 40 to a female connector not shown in the diagram.

The connector cap 10 is connected to the downstream side of the male connector 40, and is used when priming.

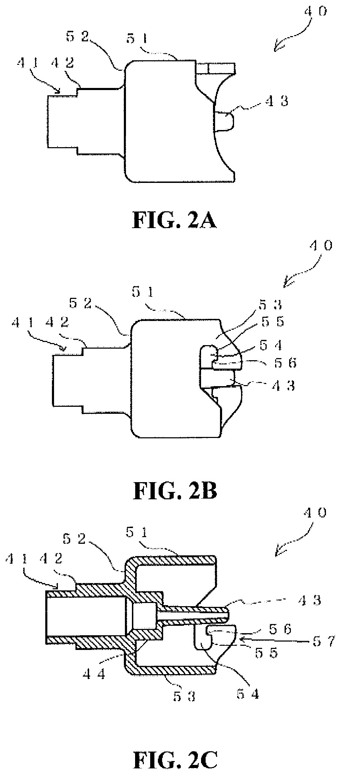

FIGS. 2A-2C are diagrams illustrating the male connector 40. FIG. 2A shows a plan view, FIG. 2B shows a side view, and FIG. 2C shows a vertical cross-sectional view.

The male connector 40 includes a circulation tube 41 which forms the flow path of the fluid such as liquid medicine, and an outer cylinder body 51 formed coaxially with the circulation tube 41 on the outside of the circulation tube 41. In the description, the left side of FIG. 2 is called the base end side (back end side), and the right side of the diagram is called the front end side.

The circulation tube 41 includes a cylindrical base tube part 42 which is connected to a catheter, syringe or the like other than the tube 5; an injection tube part 43 which is connected to the connector cap 10 or female connector (not shown in diagram); and a middle tube part 44 which connects to the base tube part 42 and injection tube part 43. The injection tube part 43 is a cannula with a tapered cross-section.

The outer cylinder body 51 includes a bottom part 52 which overhangs outwardly in a flange-like manner from the vicinity of the linking part between the base tube part 42 and middle tube part 44, and an outer cylinder part 53 which continues from the outer rim of the bottom part 52 and extends to the front end side in the axial direction and encloses the front end part of the circulation tube 41, that is, the injection tube part 43.

The front end rim of the outer cylinder part 53 includes locking parts 57 on which a groove part 54 for locking and linking the connector cap 10 or female connector (not shown in diagram) is demarcated. These locking parts 57 are formed by a locking surface 55 which responds to force in the axial direction, and a hook surface 56 which extends in a hook shape from the locking surface 55 and responds to force in the circumferential direction (the radial direction of the tube).

At least two locking parts 57 are provided at positions opposite the front end rim part of the outer tube part 53.

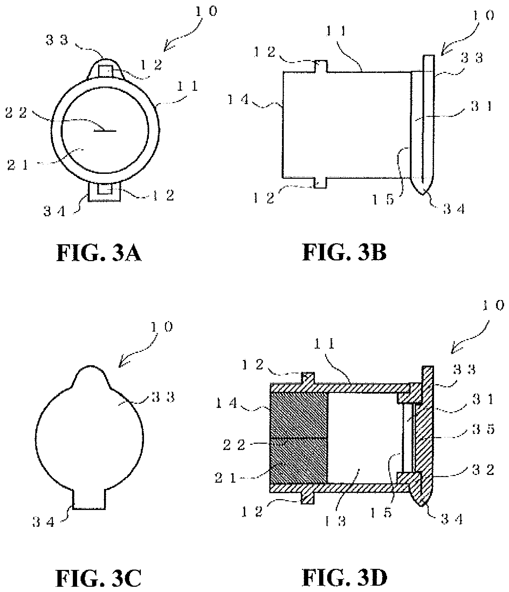

FIGS. 3A-3D are diagrams illustrating a connector cap 10 according to embodiment 1. FIG. 3A shows an elevation view, FIG. 3B shows a side view, FIG. 3C shows a rear view, and FIG. 3D shows a vertical cross-sectional view.

The connector cap 10 comprises a housing 11 formed in a substantially cylindrical shape, a septum 21 and a filter 31. The housing 11 is equivalent to the container of the present disclosure.

The housing 11 is made of synthetic resin or the like and has a substantially cylindrical shape. On the end part in the axial direction, a first opening 14 and a second opening 15 are provided. The male connector 40 is connected on the first opening 14 side of the housing 11. The interior space of the housing 11, which is the space other than that occupied by the septum 21 (described later) disposed in the first opening 14 of the housing 11, is called the reservoir 13. The reservoir 13 can store fluid such as liquid medicine, and the volume of the reservoir 13 is about 0.06 - 0.1 milliliters, for example.

On the outer circumferential surface of the housing 11, at least two protrusions 12 are provided, which can fit together with the locking parts 57 of the male connector and can couple together with the hook surface 56. The coupling surface of the protrusion 12 with the hook surface 56 of the male connector 40 is formed in a linear shape such that the two can couple together in a linear manner. In addition, protrusions 12 preferably have a shape which connects with or fits together with both the locking surface and the hook surface of the male connector 40.

The septum 21 is equivalent to the seal member of the present disclosure. It is made of an elastic material such as rubber, and is held in the first opening 14 of the housing 11. In the center part of the septum 21, a slit 22 equivalent to the insertion passage of the present disclosure is provided. The slit 22 is formed such that the injection tube part 43 of the male connector 40, equivalent to the inserted body of the present disclosure, can be inserted. The septum 21 has an elastic force by which ease of insertion of the injection tube part 43 and sealability after removal of the injection tube part 43 are both appropriately achieved. Also, the thickness of the septum 21 in the axial direction is of a degree such that when the locking parts 57 of the male connector 40 and the protrusions 12 of the connector cap 10 are in the coupled state, the slit 22 opens and fluid can flow inside the injection tube part 43 and the reservoir 13.

The filter 31 is constructed of a hydrophobic filter through which air can pass but liquid cannot. The filter 31 is held on a filter attachment part 32, and is attached to the second opening 15 of the housing 11 via the filter attachment part 32.

The filter attachment part 32 has a ring shape (donut shape) or polygonal shape which fits together with the second opening 15 of the housing 11, and it holds the filter 31 on the inner circumferential side of the filter attachment part 32. The filter attachment part 32 can be attached to and detached from the second opening 15. Therefore, it can be said that the filter 31 is attachable to and detachable from the housing 11 via the filter attachment part 32.

The filter attachment part 32 includes a cover 33 provided via a hinge 34 disposed on the outer circumferential part of the filter attachment part 32. The cover 33 can open and close by means of the hinge 34. When the cover 33 is closed, the filter 31 can be covered from the outside. That is, when the cover 33 is closed, the filter 31 is in a state where it is not exposed to the outside of the connector cap 10. On the inner surface side of the cover 33, a locking part 35 is constructed from a convex part having substantially the same external shape as the inner circumference of the filter attachment part 32. When the cover 33 is closed, a cover locking part 35 fits together with the inner circumference of the filter attachment part 32, and the outer circumference of the cover locking part 35 and the inner circumference of the filter attachment part 32 are tightly sealed, such that liquid cannot leak (liquid-tight state) and air cannot leak (air-tight state) from the second opening 15. The configuration which forms the liquid-tight state and air-tight state inside the cover 33 is not limited to this. For example, a cover locking part can be provided, which covers the second opening 15 from the outside. Also, the cover 33 according to embodiment 1 can have a button at a position opposite the hinge 34, such that it is easy to open and close the cover 33 by applying a finger to the button. The cover part of the present disclosure is equivalent to the cover 33, hinge 34 and cover locking part 35 of this embodiment.

Next, priming of the transfusion line set 1 configured as above will be described. When priming is performed, first, the connector cap 10 is connected to the male connector 40 attached to the front end of the tube 5.

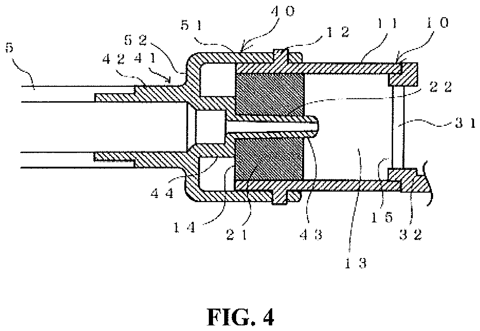

FIG. 4 is a schematic cross-sectional diagram illustrating the connected state of the male connector 40 and connector cap 10 according to embodiment 1. In FIG. 4, the cover 33 of the connector cap 10 is omitted.

When the male connector 40 and connector cap 10 are connected, first, the injection tube part 43 of the male connector 40 is brought close to the septum 21 of the connector cap 10. Then, the injection tube part 43 of the male connector 40 is inserted into the slit 22 of the septum 21 of the connector cap 10. At this time, the connector cap 10 is rotated in the circumferential direction such that the protrusions 12 of the connector cap 10 fit together with the groove part 54 of the male connector 40. When the protrusions 12 fit together with the groove part 54, the hook surface 56 of the locking parts 57 and the protrusions are connected, and the locations of the protrusions are restricted inside the groove part 54. As a result, the male connector 40 and connector cap 10 are connected in a stable state.

In the connected state shown in FIG. 4, the front end of the injection tube part 43 of the male connector 40 pierces through the septum 21 of the connector cap 10, and is in a state where it protrudes inside the reservoir 13 of the housing 11. Also, the middle tube part 44 strikes the surface on the inlet side of the septum 21. The septum 21, which is made of an elastic material, tightly adheres to the outer circumferential surface of the injection tube part 43, such that liquid does not leak between the injection tube part 43 and septum 21. In this embodiment, in the connected state, the front end of the injection tube part 43 of the male connector 40 pierces the septum 21 and protrudes inside the reservoir 13, but the front end of the injection tube 43 does not necessarily have to protrude inside the reservoir 13. Instead, the injection tube part 43 can be inserted inside the housing 11 to an extent that the slit 22 opens and fluid can flow.

Next, the priming procedure will be described.

With the cover 33 of the connector cap 10 in the open state, the technician adjusts the roller clamp 6 to allow fluid to flow inside the tube 5. In so doing, air inside the tube 5 and the flow path of the male connector 40 passes through the filter 31 and is gradually exhausted to outside the connector cap 10. The fluid that has advanced toward the downstream side inside the tube 5 infiltrates the base tube part 42 of the male connector 40, passes through the middle tube part 44, and proceeds inside the injection tube part 43. When the fluid that has come out from the front end opening of the injection tube part 43 enters the reservoir 13, air from the tube 5 and the flow path of the male connector 40 is exhausted, and therefore, the technician adjusts the roller clamp 6 and stops the flow of fluid, and priming is complete.

When priming is finished, the technician closes the cover 33 of the connector cap 10. As a result, fluid such as liquid medicine does not leak outside the connector cap 10 via the filter 31 even if the filter 31 has come into contact with a surfactant such as alcohol and become hydrophilic.

Then, the connector cap 10 is removed from the male connector 40. Specifically, by rotating the connector cap 10 circumferentially, the protrusions 12 of the connector cap 10 and the locking parts 57 of the male connector 40 are no longer secured together, and the protrusions 12 are removed from the groove part 54. Then, the injection tube part 43 of the male connector 40 is pulled out from the septum 21. As a result, the slit 22 closes due to the restoring force of the septum 21 which is made of an elastic material, and therefore, the fluid that infiltrated the reservoir 13 of the connector cap 10 during priming is sealed inside the reservoir 13, and cannot leak outside the connector cap 10.

As described above, the connector cap 10 according to embodiment 1 includes a septum 21 which blocks the reservoir 13 from the outside when the injection tube part 43 of the male connector is not inserted into the first opening 14 which opens on the inlet side of the housing 11, and it includes a hydrophobic filter 31 in the second opening 15 which opens on the outlet side of the reservoir 13. During priming, leaking of liquid medicine via the filter can be prevented because air is exhausted via the hydrophobic filter 31, but the liquid medicine is sealed inside the reservoir 13. Also, even if the connector cap 10 is removed from the male connector 40, the fluid that flowed into the reservoir 13 during priming is sealed inside the reservoir 13 by the septum 21. Therefore, leaking of liquid during priming can be prevented. Also, the amount of liquid medicine drained from the male connector 40 during priming is at most only the amount that can be accommodated inside the reservoir 13, and thus the amount of liquid medicine that goes to waste during priming can be reduced.

Also, on the outside of the filter 31 attached to the second opening 15 of the reservoir 13, there is a cover 33 which covers the filter 13 such that it is liquid-tight. As a result, by closing the cover 33 to cover the filter 31 after priming is finished, liquid medicine which has flowed into the reservoir 13 of the connector cap during priming can be prevented from seeping outside via the hydrophobic filter even if the hydrophobic filter has become hydrophilic. Also, by closing the cover 33 to cover the filter 31 after priming is finished, air is prevented from being sucked into the male connector 40 via the filter 31 after priming.

Also, in embodiment 1, there is a filter attachment part 32, which removably attaches the filter 31 to the second opening 15 of the reservoir filter 31. Since the filter 31 can be attached and detached by means of the filter attachment part 32, in the event that the hydrophobic filter becomes hydrophilic due to contact with a surfactant such as alcohol, priming can be performed after replacing only the filter. Also, a filter compatible with the characteristics of the circulated liquid, for example, can be attached to the reservoir 13, further suppressing leakage of fluid via the filter 31.

### Embodiment 2

In embodiment 2, a male connector having a circulation tube on which a luer part is formed on the front end side, and a connector cap which can be attached to it, are explained as an example. In embodiment 2, primarily the differences from embodiment 1 are described, and the same reference numerals are assigned to constituent elements which are the same as or correspond to those of embodiment 1.

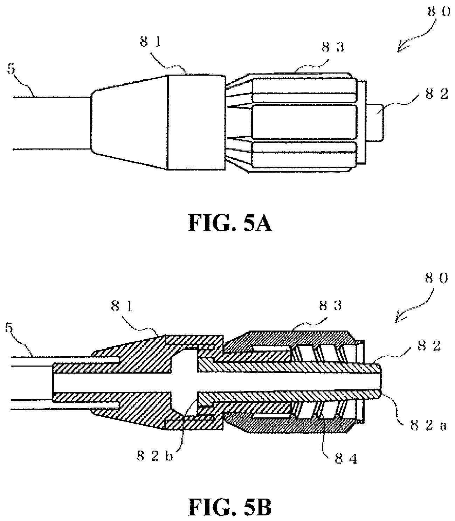

FIGS. 5A and 5B are diagrams which explain a male connector 80 according to embodiment 2. FIG. 5A shows a side view, and FIG. 5B shows a cross-sectional view. The male connector 80 includes an outer cylinder part 81 formed in a substantially cylindrical shape, a male luer part 82 provided on the front end side inside the outer cylinder part 81, and a lock ring 83 attached on the outer circumference of the front end side of the outer cylinder part 81. FIG. 5 shows the state where a tube 5 is connected to the male connector 80, and in the description, the left side of the diagram is called the base end side, and the right side of the diagram is called the front end side.

The outer cylinder part 81 is substantially cylindrical, and its diameter gradually increases in the direction from the base end side portion connected to the tube 5 to the front end side, and it extends from the front end in the forward direction. A flow path for conducting liquid medicine is formed inside the outer cylinder part 81, and its front end connects with the flow path of the male luer part 82.

The male luer part 82 is attached to the inner circumferential surface of the front end of the outer cylinder part 81 such that it is in liquid-tight contact, and in the vicinity of the front end 82a. The male luer part 82 is formed in a cylindrical shape having a gentle taper, such that the diameter becomes smaller moving toward the front end. The base end 82b of the male luer part 82 is located inside the flow path inside the outer cylinder part 81. The male luer part 82 is equivalent to the injection tube part of the present disclosure.

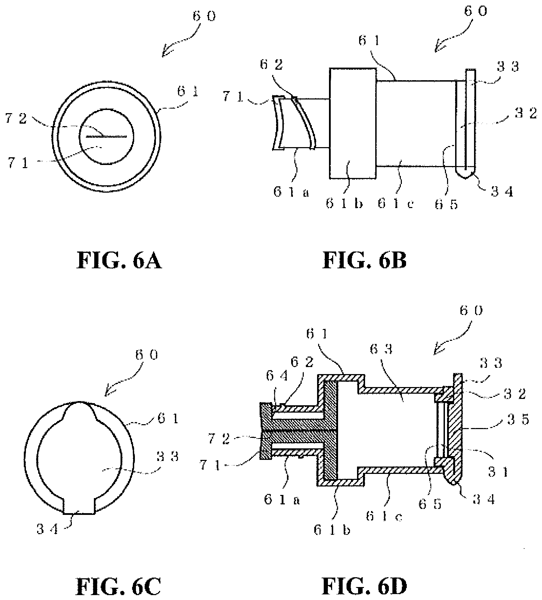

The inside of the lock ring 83 is formed into a substantially cylindrical shape, which can accommodate the connection side end part of a female connector (not shown in diagram) or a connector cap 60 (refer to FIG. 6), which is the connection end of the male connector 80. That is, the lock ring 83 is set to a size such that the connection side end part of the connector cap 60 (end part of the housing 61 on the side where the septum 71 is attached; details are described later) can fit between it and the male luer part 82. The lock ring 83 can rotate in the circumferential direction and can move in the axial direction of the male luer part 82.

Also, a female screw 84 is formed from the front end part to the center part in the axial direction on the inner circumferential surface of the lock ring 83.

FIGS. 6A-6D are diagrams showing a connector cap according to embodiment 2. FIG. 6A shows an elevation view, FIG. 6B shows a side view, FIG. 6C shows a rear view, and FIG. 6D shows a vertical cross-sectional view. The connector cap 60 comprises a housing 61 formed in a substantially cylindrical shape, a septum 71 and a filter 31. The housing 61 is equivalent to the container of the present disclosure.

The housing 61 is made of synthetic resin or the like and has a substantially cylindrical shape. On the end part in the axial direction, a first opening 64 and a second opening 65 are provided. A male connector 80 is connected on the first opening 64 side of the housing 61. The interior space of the housing 61, which is the space other than that occupied by the septum 71 (described later) disposed in the first opening 64 of the housing 61, is called the reservoir 63. The reservoir 63 can store fluid such as liquid medicine, and the volume of the reservoir 63 is about 0.06 - 0.1 milliliters, for example.

On the outer circumferential surface on the upstream side of the housing 61, that is, on the side where it is connected with the male connector 80, a coupling part 62 of the housing 61 is formed which can screw into a female screw 84. In embodiment 2, the housing 61 is constructed of three regions of different diameters, which are, in order from the upstream side, a front part 61a of small diameter, a middle part 61b of larger diameter than that of the front part 61a, and a back end part 61c of diameter larger than that of the front part 61a and smaller than that of the middle part 61b. Although the shape of the housing 61 is not limited to this, in embodiment 2, the inner wall surface of the housing 61 includes inner diameters corresponding to the diameters of the front part 61a, middle part 61b and back end part 61c. The septum 71 is positioned within the housing 61 according to these inner diameters.

The septum 71 is equivalent to the seal member of the present disclosure. It is made of an elastic material such as rubber, and is disposed in the first opening 64 of the housing 61. In the center part of the septum 71, a slit 72 equivalent to the insertion passage of the present disclosure is provided. The slit 72 is formed such that the male luer part 82 of the male connector 80 as the inserted body of the present disclosure can be inserted. The septum 71 has an elastic force by which ease of insertion of the male luer part 82 and sealability after removal of the male luer part 82 are both appropriately achieved. The thickness of the septum 71 in the axial direction is of a degree such that when the female screw 84 of the male connector 80 and the coupling part 62 of the connector cap 60 are in the coupled state, the slit 72 opens and fluid can flow between the male luer part 82 and the reservoir 63.

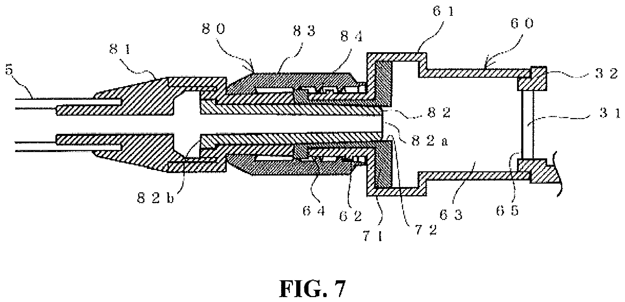

FIG. 7 is a schematic cross-sectional diagram illustrating the connected state of the male connector 80 and connector cap 60 according to embodiment 2. In FIG. 7, the cover 33 of the connector cap 60 is omitted.

When the male connector 80 and connector cap 60 are connected, first, the front end opening of the male luer part 82 of the male connector 80 is brought close to the septum 71 of the connector cap 60. Then, the male luer part 82 of the male connector 80 is inserted into the slit 72 of the septum 71 of the connector cap 60. Then, the female screw 84 of the lock ring 83 and the coupling part 62 of the connector cap 60 are brought into contact with each other, after which the lock ring 83 is rotated in a specified circumferential direction, and as a result, the female screw 84 and coupling part 62 are screwed together.

Then, when the female screw 84 and coupling part 62 are screwed together in an appropriate state, as shown in FIG. 7, the slit 72 of the septum 71 is spread wide, making it possible for fluid to flow between the male luer part 82 and the inside of the reservoir 63 of the connector cap 60. In the connected state shown in FIG. 7, the front end of the male luer part 82 is not in a state where it pierces through the septum 71 and protrudes inside the reservoir 63, but it is in a state where it is inserted inside the housing 61. The septum 71, which is made of an elastic material, tightly adheres to the outer circumferential surface of the male luer part 82, such that liquid does not leak between the male luer part 82 and the septum 71. In this embodiment, in the connected state, the front end of the male luer part 82 does not protrude inside the reservoir 63, but it is also possible that the front end of the male luer part 82 does protrude inside the reservoir 63.

When the male connector 80 and connector cap 60 configured in this way are used, priming can be performed by the same procedure as in embodiment 1, and the same effect can be obtained.

### Embodiment 3

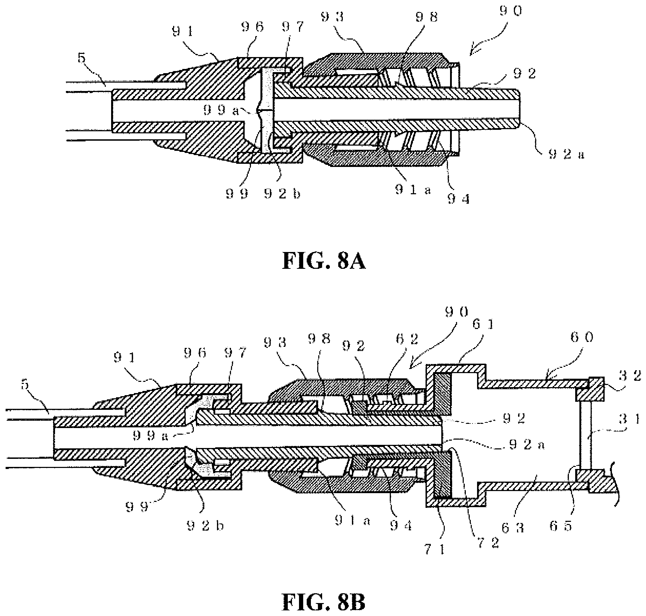

Referring to FIGS. 8A and 8B, a male connector 90 according to embodiment 3 comprises a valve which is open when connected with the connector cap or female connector, and is closed when not connected. In embodiment 3, primarily the differences from embodiment 2 are described, and the same reference numerals are assigned to constituent elements which are the same as or correspond to those of embodiment 2.

FIGS. 8A and 8B are schematic cross-sectional diagrams of a male connector 90 and connector cap 60 according to embodiment 3. FIG. 8A shows the state where a valve 99 of the male connector 90 is closed, and FIG. 8B shows the state where the connector cap 60 is connected to the male connector 90 and the valve 99 is open. In FIGS. 8A and 8B, the left side of the diagram is called the base end side, and the right side of the diagram is called the front end side.

The male connector 90 comprises an outer cylinder part 91 which is substantially cylindrical, a male luer part 92 provided on the front end side inside the outer cylindrical part 91, and a lock ring 93 attached on the outer circumference of the front end side of the outer cylindrical part 91.

The outer cylinder part 91 is substantially cylindrical, and its diameter gradually increases in the direction from the base end side portion connected to the tube 5 to the front end, and it extends from the front end in the forward direction. A flow path for filling liquid medicine is formed inside the outer cylinder part 91, and its front end connects with the flow path of the male luer part 92. The flow path inside the outer cylinder part 91 is formed from a large-diameter part 96 and a small-diameter part 97, which has a smaller diameter than that of the large-diameter part 96. That is, the flow path inside the outer cylinder part 91 is divided into at least two parts of different diameters, a large-diameter part 96 and a small-diameter part 97.

The valve 99 is attached in a state where the rim has been inserted between the large-diameter part 96 and small-diameter part 97. That is, the valve 99 is attached such that the rim of the valve 99 is inserted between the large-diameter part 96 and small-diameter part 97, separating the large-diameter part 96 and small-diameter part 97. In substantially the center position of the valve 99, an expansion/contraction hole 99a is formed, which opens and closes by expansion and contraction.

The male luer part 92 is attached to the inner circumferential surface of the front end of the outer cylinder part 91 such that it is in liquid-tight contact. The male luer part 92 is formed in a cylindrical shape having a gentle taper in the vicinity of the front end 92a such that the diameter becomes smaller moving toward the front end. The male luer part 92 is equivalent to the injection tube part of the present disclosure.

In the center in the axial direction of the outer circumferential part of the male luer part 92, a position restricting protrusion 98 is provided, which overhangs outwardly in a flange-like manner.

The male luer part 92 can move in the axial direction, and as shown in FIG. 8A, when in the state where it has moved to the front end side in the axial direction (front end 92a side), the expansion/contraction hole 99a of the valve 99 is in the closed state. When the male luer part 92 moves to the base end side in the axial direction (base end 92b side), the base end 92b of the male luer part 92 presses on the valve 99, and the valve 99 opens. Movement of the male luer part 92 toward the base end side is restricted by the fact that the position restricting protrusion 98 contacts the front end part 91a of the outer cylinder part 91. That is, in embodiment 3, movement of the male luer part 92 in the axial direction is restricted within a specified range by the front end part 91a of the outer cylinder part 91 and the position restricting protrusion 98 of the male luer part 92.

The inside of the lock ring 93 is formed into a substantially cylindrical shape, which can accommodate the connection side end part of a female connector (not shown in diagram) or the connector cap 60, which is the connection end of the male connector 90. That is, the lock ring 93 is set to a size such that the connection side end part of the connector cap 60 can fit between it and the male luer part 82. The lock ring 93 can rotate in the circumferential direction and can move in the axial direction of the male luer part 92.

Also, a female screw 94 is formed from the front end part to the center portion in the axial direction on the inner circumferential surface of the lock ring 93.

Connection of the male connector 90 and connector cap 60 configured in this way will be explained. When the male connector 90 and connector cap 60 are connected, first, the front end opening of the male luer part 92 of the male connector 90 and the septum 71 of the connector cap 60 are brought close to each other. Then, the male luer part 92 of the male connector 90 is inserted into the slit 72 of the septum 71 of the connector cap 60. Then, the female screw 94 of the lock ring 93 and the coupling part 62 of the connector cap 60 are brought into contact with each other, after which the lock ring 93 is rotated in a specified circumferential direction, and as a result, the female screw 94 and coupling part 62 are screwed together.

When the male luer part 92 penetrates the septum 71 of the connector cap 60, the male luer part 92 is pressed toward the base end 92b side. Then, when the female screw 94 and coupling part 62 are screwed together in an appropriate state, the male luer part 92 moves to the base end side in the axial direction (base end 92b side), and the base end 92b of the male luer part 92 presses on the valve 99, causing the expansion/contraction hole 99a of the valve 99 to open.

When priming is performed, it can be performed in this state.

When priming is finished, the connector cap 60 is removed from the male connector 90. As a result, the pressure of the male luer part 92 applied toward the base end 92b side is released, and due to the restoring force (elastic force) which restores the shape of the valve 99, the male luer part 92 moves in the direction of the front end 92a, and the valve 99 closes. For this reason, liquid medicine or the like does not leak from the front end of the male luer part 92 even after the connector cap 60 is removed after priming.

According to embodiment 3 as described above, priming can be performed by the same procedure as in embodiment 2, and the same effect can be obtained.

In addition, the male connector 90 according to embodiment 3 may include a valve 99 which is closed when the male luer part 92 moves to the front end side in the axial direction, and is opened when the male luer part 92 moves to the base end side in the axial direction. As a result, leakage of liquid medicine or the like from the male connector 90 side can be prevented when the connector cap 60 is removed after priming.

Although the illustrative embodiments of the present disclosure have been described herein with reference to the accompanying drawings, the above description, disclosure, and figures should not be construed as limiting, but merely as exemplifications of particular embodiments.

## Claims

1. A connector cap (10, 60) which can be removably attached to a male connector (40, 80, 90), comprising:
a container (11, 61) including a first opening (14, 64) and a second opening (15, 65) and having an inside and an outside;
a seal member (21, 71) disposed in the first opening (14, 64) so as to separate the inside and the outside of the container (11, 61), the seal member (21, 71) including an insertion passage (22, 72) formed therethrough whereby an inserted body (43, 82, 92) can be inserted from the outside to the inside of the container (11, 61); a hydrophobic filter (31) which is disposed in the second opening (15, 65); the connector cap being **characterized in that** the seal member (21, 71) has an elastic force by which ease of insertion of the inserted body (43, 82, 92) and sealabilty after removal of the inserted body (43, 82, 92) are both achieved.

2. The connector cap (10, 60) according to claim 1, further comprising a cover (33) which can cover the second opening (15, 65) in a liquid-tight state from the outside of the hydrophobic filter (31).

3. The connector cap (10, 60) according to any of the preceding claims, further comprising a filter attachment (32) which removably attaches the hydrophobic filter (31) to the second opening (15, 65).

4. The connector cap (10, 60) according to any of the preceding claims, wherein the seal member (21, 71) is a septum seal.

5. The connector cap (10, 60) according to any of the preceding claims, wherein the inside of the container (11, 61) defines a reservoir (13, 63) for storing fluid.

6. The connector cap (10, 60) according to claim 5, wherein the volume of the reservoir (13, 63) is about 0.06 to about 0.1 milliliters.

7. A transfusion line connection apparatus (1) comprising:
a connector cap (10, 60) according to any of the preceding claims; and
a male connector (40, 80, 90) having an injection tube (43, 82, 92) which can be inserted into the insertion passage (22, 72) of the seal member (21, 71) of the connector cap (10, 60).

8. A transfusion line connection apparatus (1) according to claim 7, wherein the connector cap (10, 60) includes at least two protrusions (12) disposed on the outside of the container (10) for coupling to the male connector (40, 80, 90).

9. A transfusion line connection apparatus (1) according to claim 8, wherein the male connector (40, 80) includes at least two locking parts (57), the locking parts (57) adapted to receive the at least two protrusions (12).

10. A transfusion line connection apparatus (1) according to claim 7, the transfusion line connection apparatus (1) further including a lock ring (83, 93) attachable to the male connector (40, 80, 90), the lock ring (83, 93) including a female screw (84, 94) formed on an inner surface.

11. A transfusion line connection apparatus (1) according to claim 10, wherein the connector cap (10, 60) includes a coupling part (62) disposed on an outer surface and adapted to screw (84, 94) into the female screw of the lock ring (83, 93).

12. A transfusion line connection apparatus (1) according to any of claims 7-11, wherein the container (11, 61) is constructed of three regions having different diameters including a front part (61a), a middle part (61b), and a back end part (61c).

13. A transfusion line connection apparatus (1) according claim 12, wherein the back end part (61c) has a larger diameter than the front part (61a).

14. A transfusion line connection apparatus (1) according any of claims 12-13, wherein the middle part (61b) has a larger diameter than the front part (61a) and the back end part (61c).

15. A transfusion line connection apparatus (1) according to any of claims 7-14, wherein the male connector (40, 80, 90) defines a large diameter part (96) and a small diameter part (97) and includes a valve (99) having a rim inserted between the large diameter part (96) and the small diameter part (97).

## Patentansprüche

1. Verschlusskappe (10, 60), welche lösbar an einem Stecker-Verschluss (40, 80, 90) befestigt werden kann, umfassend:
einen Behälter (11, 61), welcher eine erste Öffnung (14, 64) und eine zweite Öffnung (15, 65) umfasst und einen Innenraum und eine Außenseite aufweist;
ein Abdichtglied (21, 71), welches in der ersten Öffnung (14, 64) so angeordnet ist, dass es den Innenraum und die Außenseite des Behälters (11, 61) voneinander trennt, wobei das Abdichtglied (21, 71) einen Einführdurchgang (22, 72) umfasst, welcher durch dasselbe geformt ist, wodurch ein eingeführter Körper (43, 82, 92) von der Außenseite her in den Innenraum des Behälters (11, 61) eingeführt werden kann; einen hydrophoben Filter (31), welcher in der zweiten Öffnung (15, 65) angeordnet ist; wobei die Verschlusskappe **dadurch gekennzeichnet ist, dass** das Abdichtglied (21, 71) eine elastische Kraft aufweist, durch welche sowohl eine einfache Einführung des eingeführten Körpers (43, 82, 92) als auch eine Abdichtungsfähigkeit nach der Abnahme des eingeführten Körpers (43, 82, 92) erreicht werden.

2. Verschlusskappe (10, 60) nach Anspruch 1, ferner umfassend eine Abdeckung (33), welche die zweite Öffnung (15, 65) in einem flüssigkeitsdichten Zustand von der Außenseite des hydrophoben Filters (31) abdecken kann.

3. Verschlusskappe (10, 60) nach einem der vorhergehenden Ansprüche, ferner umfassend eine Filterbefestigung (32), welche den hydrophoben Filter (31) lösbar an der zweiten Öffnung (15, 65) befestigt.

4. Verschlusskappe (10, 60) nach einem der vorhergehenden Ansprüche, wobei das Abdichtglied (21, 71) eine Scheidewanddichtung ist.

5. Verschlusskappe (10, 60) nach einem der vorhergehenden Ansprüche, wobei der Innenraum des Behälters (11, 61) ein Reservoir (13, 63) zum Speichern von Fluiden definiert.

6. Verschlusskappe (10, 60) nach Anspruch 5, wobei das Volumen des Reservoirs (13, 63) etwa 0,06 bis etwa 0,1 Milliliter beträgt.

7. Infusionsschlauchverbindungsvorrichtung (1), umfassend:
eine Verschlusskappe (10, 60) nach einem der vorhergehenden Ansprüche; und
einen Stecker-Verschluss (40, 80, 90), welcher einen Injektionsschlauch (43, 82, 92) aufweist, welcher in den Einführdurchgang (22, 72) des Abdichtglieds (21, 71) der Verschlusskappe (10, 60) eingeführt werden kann.

8. Infusionsschlauchverbindungsvorrichtung (1) nach Anspruch 7, wobei die Verschlusskappe (10, 60) zumindest zwei Vorsprünge (12) umfasst, welche auf der Außenseite des Behälters (10) zum Eingriff mit dem Stecker-Verschluss (40, 80, 90) angeordnet sind.

9. Infusionsschlauchverbindungsvorrichtung (1) nach Anspruch 8, wobei der Stecker-Verschluss (40, 80) zumindest zwei Verriegelungsteile (57) umfasst, wobei die Verriegelungsteile (57) ausgebildet sind, um die zumindest zwei Vorsprünge (12) aufzunehmen.

10. Infusionsschlauchverbindungsvorrichtung (1) nach Anspruch 7, wobei die Infusionsschlauchverbindungsvorrichtung (1) ferner einen Verriegelungsring (83, 93) umfasst, welcher an dem Stecker-Verschluss (40, 80, 90) befestigbar ist, wobei der Verriegelungsring (83, 93) ein Innengewinde (84, 94) umfasst, welches auf einer Innenfläche gebildet ist.

11. Infusionsschlauchverbindungsvorrichtung (1) nach Anspruch 10, wobei die Verschlusskappe (10, 60) einen Kopplungsteil (62) umfasst, welcher auf einer Außenfläche angeordnet ist und ausgebildet ist, um in das Innengewinde des Verriegelungsrings (83, 93) eingeschraubt zu werden (84, 94).

12. Infusionsschlauchverbindungsvorrichtung (1) nach einem der Ansprüche 7-11, wobei der Behälter (11, 61) aus drei Bereiche besteht, welche unterschiedliche Durchmesser aufweisen, umfassend einen Vorderteil (61a), einen Mittelteil (61b), und einen Rückendteil (61c).

13. Infusionsschlauchverbindungsvorrichtung (1) nach Anspruch 12, wobei der Rückendteil (61c) einen Durchmesser aufweist, welcher größer als der des Vorderteils (61a) ist.

14. Infusionsschlauchverbindungsvorrichtung (1) nach einem der Ansprüche 12-13, wobei der Mittelteil (61b) einen größeren Durchmesser aufweist, als der des Vorderteils (61a) und des Rückendteils (61c).

15. Infusionsschlauchverbindungsvorrichtung (1) nach einem der Ansprüche 7-14, wobei der Stecker-Verschluss (40, 80, 90) einen Teil (96) großen Durchmessers und einen Teil (97) kleinen Durchmessers definiert, und wobei er ein Ventil (99) umfasst, welches einen Rand aufweist, welcher zwischen dem Teil (96) großen Durchmessers und dem Teil (97) kleinen Durchmessers eingeführt ist.

## Revendications

1. Capuchon de connecteur (10, 60) pouvant être fixé de manière amovible sur un connecteur mâle (40, 80, 90), comprenant :
un récipient (11, 61) incluant une première ouverture (14, 64) et une deuxième ouverture (15, 65) et comportant un intérieur et un extérieur ;
un élément d'étanchéité (21, 71) disposé dans la première ouverture (14, 64), de sorte à séparer l'intérieur et l'extérieur du récipient (11, 61), l'élément d'étanchéité (21, 71) incluant un passage d'insertion (22, 72) le traversant, un corps inséré (48, 82, 92) pouvant ainsi être inséré de l'extérieur vers l'intérieur du récipient (11, 61) ; un filtre hydrophobe (31) disposé dans la deuxième ouverture (15 ; 65) ; le capuchon de connecteur étant **caractérisé en ce que** l'élément d'étanchéité (21, 71) présente une force élastique, assurant la facilité d'insertion du corps inséré (43, 82, 92) et la capacité d'établissement de l'étanchéité après le retrait du corps inséré (43, 82, 92).

2. Capuchon de connecteur (10, 60) selon la revendication 1, comprenant en outre un couvercle (33) pouvant recouvrir la deuxième ouverture (15, 65) dans un état étanche aux liquides à partir de l'extérieur du filtre hydrophobe (31).

3. Capuchon de connecteur (10, 60) selon l'une quelconque des revendications précédentes, comprenant en outre une fixation de filtre (32) fixant de manière amovible le filtre hydrophobe (31) sur la deuxième ouverture (15, 65).

4. Capuchon de connecteur (10, 60) selon l'une quelconque des revendications précédentes, dans lequel l'élément d'étanchéité (21, 71) est un joint à septum.

5. Capuchon de connecteur (10, 60) selon l'une quelconque des revendications précédentes, dans lequel l'intérieur du récipient (11, 61) définit un réservoir (13, 63) pour stocker un fluide.

6. Capuchon de connecteur (10, 60) selon la revendication 5, dans lequel le volume du réservoir (13, 63) est compris entre environ 0,06 et environ 0,1 millilitres.

7. Appareil de connexion de ligne de transfusion (1), comprenant :
un capuchon de connecteur (10, 60) selon l'une quelconque des revendications précédentes ; et
un connecteur mâle (40, 80, 90) comportant un tube d'injection (43, 82, 92) pouvant être inséré dans le passage d'insertion (22, 72) de l'élément d'étanchéité (21, 71) du capuchon de connecteur (10, 60).

8. Appareil de connexion de ligne de transfusion (1) selon la revendication 7, dans lequel le capuchon de connecteur (10, 60) inclut au moins deux protubérances (12) disposées sur l'extérieur du récipient (10) pour l'accouplement du connecteur mâle (40, 80, 90).

9. Appareil de connexion de ligne de transfusion (1) selon la revendication 8, dans lequel le connecteur mâle (40, 80) inclut au moins deux parties de verrouillage (57), les parties de verrouillage (57) étant adaptées pour recevoir les au moins deux protubérances (12).

10. Appareil de connexion de ligne de transfusion (1) selon la revendication 7, l'appareil de connexion de la ligne de transfusion (1) incluant en outre une bague de verrouillage (83, 93) pouvant être fixée sur le connecteur mâle (40, 80, 90), la bague de verrouillage (83, 93) incluant une vis femelle (84, 94) formée sur une surface interne.

11. Appareil de connexion de ligne de transfusion (1) selon la revendication 10, dans lequel le capuchon de connecteur (10, 60) inclut une partie d'accouplement (62) disposée sur une surface externe et adaptée pour un vissage (84, 94) dans la vis femelle de la bague de verrouillage (83, 93).

12. Appareil de connexion de ligne de transfusion (1) selon l'une quelconque des revendications 7 à 11, dans lequel le récipient (11, 61) comprend trois régions ayant des diamètres différents, incluant une partie avant (61a), une partie médiane (61b) et une partie d'extrémité arrière (61c).

13. Appareil de connexion de ligne de transfusion (1) selon la revendication 12, dans lequel la partie d'extrémité arrière (61c) a un diamètre supérieur à celui de la partie avant (61a).

14. Appareil de connexion de ligne de transfusion (1) selon l'une quelconque des revendications 12 à 13, dans lequel la partie médiane (61b) a un diamètre supérieur à ceux de la partie avant (61a) et de la partie d'extrémité arrière (61c).

15. Appareil de connexion de ligne de transfusion (1) selon l'une quelconque des revendications 7 à 14, dans lequel le connecteur mâle (40, 80, 90) définit une partie à grand diamètre (96) et une partie à diamètre réduit (97), et inclut une valve (99) comportant un rebord insérée entre la partie à grand diamètre (96) et la partie à diamètre réduit (97).
